# EUROPEAN PATENT SPECIFICATION

(11) **EP 4 320 108 B1**
(45) Date of publication and mention of the grant of the patent: **30.10.2024**
(21) Application number: 22722695.8
(22) Date of filing: 08.04.2022
(51) Int. Cl.: C07D 307/12, C07C 29/141, C07C 31/22, C07D 307/42, C07D 307/46

(54) **NIKEL- AND CERIUM-CATALYZED REDUCTION OF HYDROXYMETHYLFURFURAL**
NIKEL- UND CER-KATALYSIERTE REDUKTION VON HYDROXYMETHYLFURFURAL
RÉDUCTION DE L'HYDROXYMÉTHYLFURFURAL CATALYSÉE PAR LE NICKEL ET LE CÉRIUM

(30) Priority: 08.04.2021 LU 102760
(43) Date of publication of application: 14.02.2024
(73) Proprietor: National Institute of Chemistry, 1000 Ljubljana (SI)
(72) Inventor: POMEROY, Brett, 1000 Ljubljana (SI); LIKOZAR, Blaz, 4000 Kranji (SI); GRILC, Miha, 1291 Skofljica (SI)
(74) Representative: Mewburn Ellis LLP
(86) International application number: PCT/EP2022/059475
(87) International publication number: WO 2022/214674

(56) References cited:
- SHUO CHEN ET AL: "How Catalysts and Experimental Conditions Determine the Selective Hydroconversion of Furfural and 5-Hydroxymethylfurfural", CHEMICAL REVIEWS, vol. 118, no. 22, 28 November 2018 (2018-11-28), US, pages 11023 - 11117, XP055592643, ISSN: 0009-2665, DOI: 10.1021/acs.chemrev.8b00134
- LI H ET AL: "A Ce-promoted Ni-B amorphous alloy catalyst (Ni-Ce-B) for liquid-phase furfural hydrogenation to furfural alcohol", MATERIALS LETTERS, ELSEVIER, AMSTERDAM, NL, vol. 58, no. 22-23, 1 September 2004 (2004-09-01), pages 2741 - 2746, XP004524849, ISSN: 0167-577X, DOI: 10.1016/J.MATLET.2004.04.003

## Description

### Field of the Invention

The present invention relates to methods of making 2,5-bis(hydroxymethyl)furan and/or 2,5-bis(hydroxymethyl)tetrahydrofuran and/or 1 ,2,6-hexanetriol and particularly to catalysed reactions of hydroxymethylfurfural.

### Background

Considering the worldwide challenges regarding climate change, pollution, and the ever growing global energy consumption as a result from the current use of non-renewable fossil fuels, more sustainable alternatives have been extensively developed that shift us towards more renewable feedstocks.

2,5-bis(hydroxymethyl)tetrahydrofuran (BHMTHF) has been considered to be utilized in the polymer industry (similarly to other diols) as a bio-based replacement for the manufacturing of polyesters and heat insulating polymers, which are currently made exclusively from petroleum-based feedstocks.

1 ,2,6-hexanetriol (1 ,2,6-HT) has several applications including as a moisturizing agent (cremes), solvent (pharmaceuticals) and as a cross-linker in polymer manufacturing. It is also used in the production of alkyd resins, celluloid, polyurethane and synthetic rubber. Additionally, 1 ,2,6-HT is a direct intermediate for the production of 1 ,6-hexanediol. C₄-C₆ α,ω-diols, such as 1 ,6-hexanediol, are approximately a $7 billion per year global market which are commonly utilized to manufacture paints, coatings, plastics, and adhesives. Specifically, 1 ,6-hexanediol has a global market value approximately $902 million in 2019 and is sold for approximately 4400 USD per metric ton with an annual market size of 138000 tons. Currently, 1 ,6-hexanediol is produced from non-renewable resources, specifically benzene, with relatively low conversions and requires corrosive nitric acid and emits undesirable NOx emissions.

| | |
|---|---|
| 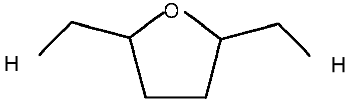 | 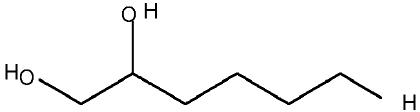 |
| 2,5-bis(hydroxymethyl)tetrahydrofuran | 1,2,6-hexanetriol |

Each of these compounds, along with many others, can be made from a starting material hydroxymethylfurfural (HMF):

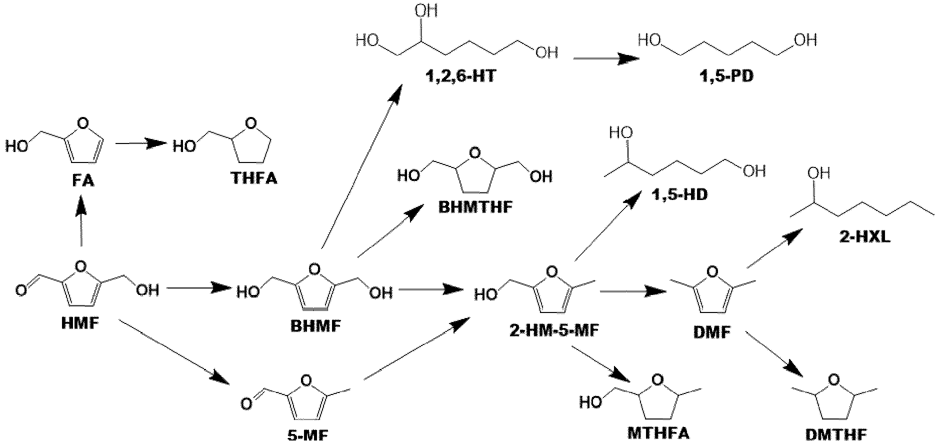

(HMF = hydroxymethylfurfural, BHMF = 2,5-bis(hydroxymethyl)furan, BHMTHF = 2,5-bis(hydroxymethyl)tetrahydrofuran, 5-MF= 5-methylfurfural, 1,2,6-HT = 1,2,6-hexanetriol, FA = Furfuryl alcohol, THFA = tetrahydrofurfuryl alcohol, 2-HM-5-MF = 5-methylfurfuryl alcohol, MTHFA = Tetrahydro-5-methylfuran-2-methanol, DMF = dimethylfuran, DMTHF = dimethyltetrahydrofuran, 1,5-HD = 1,5-hexanediol, 2-HXL = 2-hexanol, 1,5-PD = 1,5-pentadiol)

It is known that hydrogenation reactions of HMF, of the type illustrated above, can be catalysed. Kong, X et al., RSC Adv., 2014, 4, 60467-60472 describes a Raney Nickel type catalyst (metal loading >93%) to convert HMF to BHMTHF with high yields at mild temperatures of 100°C. However, the reaction relied on 1,4-dioxane as the solvent which is toxic and undesirable, as well as requiring an extremely long reaction time of 30 hours and large catalyst to HMF ratio (1:3) to achieve the desired yields.

Alamillo, R. et al, Green Chem., 2012, 14, 1413-1419 describes use of 1 wt.% Ru supported on a CeO support. However significant amounts of catalyst and a high reaction time were necessary, along with complex solvent systems.

WO 2006/063287 describes a method for the production of BHMTHF from HMF using a zirconium-supported catalyst with very high nickel loadings of 65 wt.%. US 2007/0287845 describes methods using catalysts comprising at least one metal selected from Ni, Co, Cu, Pd, Pt, Ru, Ir, Re and Rh. Shuo Chen et al., Chem. Rev., 2018, 118 (22), 11023-11117 describes various catalysts and processes to reduce furfural and HMF to various products.

Li H. et. al., Mater. Lett., 2004, 58, 22-23 teaches the reduction of furfural to furfuryl alcohol under a hydrigen atmosphere using Ni/Ce catalysis.

There remains a need for catalysed reactions of HMF to form useful products without the need for harsh reaction or solvent conditions; and in particular a method which allows tunability to select desired products or even sterochemistries of products.

The present invention has been devised in the light of the above considerations.

### Summary of the Invention

At its broadest, the present invention relates to methods of reacting HMF in the presence of Ni and Ce; the reactions of HMF may include reductions and hydrogenations. In particular, a bimetallic NiCe catalyst has been found which is particularly effective in promoting reaction of HMF; most particularly to form BHMTHF and 1,2,6-HT, and also BHMF.

The present inventors have also found that the reaction can be tuned to preferentially form one or more of BHMTHF, 1,2,6-HT and BHMF. Accordingly the present invention provides a method of making one or more of those compounds starting from HMF.

In general the HMF is in a liquid phase; suitably within a solvent. Similarly the catalyst is generally solid and hence heterogenous.

The HMF used can come from any source; using naturally or biologically derived HMF allows the present process to be highly sustainable. The present catalysts permit the use of gentle reaction conditions and solvents, including water and THF.

The present reactions can also be conducted in, for example, batch stirred slurry reactors or continuous fixed-bed reactors, permitting an efficient industrial process. That is, the catalytic process can be performed in a continuous reactor with fixed catalyst bed, or in a batch, semi-batch, or continuous stirred tank (slurry) reactor set-up with dispersed catalyst or a catalyst in a rotating basket where the catalyst is simply removed by filtration.

The present process avoids the need for noble metals, high transition metal loading, high pressure or high temperature as in the prior art.

A first aspect of the invention thus provides a method of producing 2,5-bis(hydroxymethyl)furan and/or 2,5-bis(hydroxymethyl)tetrahydrofuran and/or 1 ,2,6-hexanetriol; the method comprising the step of (a) contacting hydroxymethylfurfural, preferably in the liquid phase, with a catalyst, preferably in the solid phase, comprising Ni and Ce in an atmosphere comprising Hz at a temperature in the range 100 to 250°C; the content of Ni in the catalyst being 1-30 wt% and the content of Ce in the catalyst being 1-30 wt%. In some preferred embodiments the content of Ni in the catalyst is 5-10 wt% and the content of Ce in the catalyst is 10-15 wt%.

The present method is described in the first aspect as being "a method of producing 2,5-bis(hydroxymethyl)furan and/or 2,5-bis(hydroxymethyl)tetrahydrofuran and/or 1,2,6-hexanetriol..."; it might equally be described as a "a method of reacting HMF..." or "a method or reducing HMF..." or "a method of hydrogenating HMF...". As explained and demonstrated below, it may be the case that not all of BHMTHF, BHMF and 1,2,6-HT are formed in a given reaction according to the present invention. Only one of them may be formed, or only two of them.

The catalyst itself may be in the form of a metal oxide, ideally porous, having Ni and Ce supported thereon. In some embodiments, the porous metal oxide may be selected from Al₂O₃, SiO₂, CeO₂, BaO, CaO, MgO, ZrO₂, or TiO₂, with the preferred porous metal oxides being Al₂O₃ and SiO₂; and the most preferred being γ-Al₂O₃.

The catalyst may, as the Ni and Ce component, comprise bimetallic NiCe. This may be formed by sequential deposition of Ni and Ce (any order although preferably Ce first then Ni) onto a porous metal oxide as mentioned above.

A second aspect of the present invention therefore provides a catalytic material comprising a metal oxide, ideally porous, having Ni and Ce supported thereon; the content of Ni in the catalytic material as a whole being 1-30 wt%, preferably 5-10 wt%, and the content of Ce in the catalytic material as a whole being 1-30 wt%, preferably 10-15 wt%. In some embodiments, the porous metal oxide may be selected from Al₂O₃, SiO₂, CeO₂, BaO, CaO, MgO, ZrO₂, or TiO₂, with the preferred porous metal oxides being Al₂O₃ and SiO₂; and the most preferred being γ-Al₂O₃.

The catalyst may, as the Ni and Ce component, comprise bimetallic NiCe. This may be formed by sequential deposition of Ni and Ce (any order although preferably Ce first then Ni) onto a porous metal oxide as mentioned above.

As to reaction conditions, in some embodiments step (a) is conducted at a temperature in the range 120 to 200°C. By using a temperature in the range 160 to 200°C, formation of BHMTHF can be favoured. On the other hand, using a temperature in the range 120 to 150°C can favour formation of BHMF.

Step (a) may suitably be conduced in an H₂ atmosphere; of course, atmospheres containing sufficient Hz for hydrogenation to proceed are still useful. The atmosphere may be, for example, 50 vol% or more H₂, for example 70 vol% or more, 80 vol% or more, or 90 vol% or more. 100 vol% H₂ (pure H₂) atmosphere may be most preferred.

Step (a) can suitably be conducted at a pressure in the range 1-20 MPa, preferably 4-10 MPa. The reaction of step (a) may be allowed to proceed for, for example, 1-24 hours. The inventors have found that reaction times of 1 to 6 hours may be suitable.

As mentioned above, the HMF may be reacted in the liquid phase; in such a case, it may be present in a mixture comprising HMF and a solvent. Any inert solvent which is miscible with HMF may be suitable, but tetrahydrofuran (THF), water, 1-butanol and mixtures of two or more of these are preferred. In particular, THF or a mixture of THF and water are preferred. This mixture with HMF may be monophasic or biphasic.

Where the solvent is present, it may preferably comprise 0-50 wt% of water, and preferably is tetrahydrofuran mixed with 5-15 wt% water.

In some embodiments, the solvent and hydroxymethylfurfural mixture further comprises CaO. This has been found to encourage production of 1,2,6-HT.

The methods of the present invention may comprise further steps beyond the above step (a). For example, the method may comprise a step (b) of separating the catalyst from the liquid phase. Such a method may also comprise a further step (c) of isolating BHMF and/or BHMTHF and/or 1,2,6-HT (whichever ones have been synthesised) from the liquid phase by evaporation of any solvent used.

The invention includes the combination of the aspects and preferred features described except where such a combination is clearly impermissible or expressly avoided. The scope of the invention is defined in the appended claims.

### Summary of the Figures

Embodiments and experiments illustrating the principles of the invention will now be discussed with reference to the accompanying figures in which:
**Figure 1** shows a summary of product selectivity of all activity tests reported in Examples 1-8.
**Figure 2** is a GC-FID chromatogram of obtained products; from a reaction over NiCe/ Spheralite in 5 vol.% water in THF under 50 bar Hz atmosphere at 190 °C after 1 hour. This corresponds to Example 2, with a shortened reaction time.
**Figure 3** is a GC-FID chromatogram of obtained products; from a reaction over NiCe/ Spheralite (where ceria was calcined at 800 °C in N₂) in 5 vol.% water in THF under 50 bar Hz atmosphere. This corresponds to Example 5.
**Figure 4** is a GC-FID chromatogram of obtained products; from a reaction over NiCe/ Spheralite in 5 vol.% water in THF under 50 bar Hz atmosphere with the addition of CaO. This corresponds to Example 4.

### Detailed Description of the Invention

Aspects and embodiments of the present invention will now be discussed with reference to the accompanying figures. Further aspects and embodiments will be apparent to those skilled in the art.

The present methods involve reaction of HMF under a reducing or hydrogenating atmosphere. The subsequent reactions of HMF are catalysed by a Ni (nickel) and Ce (cerium) containing catalyst, as described in more detail below.

### Reactant Mixture

In the main step of the present methods, herein referred to as step (a), HMF is used as the primary reactant. That HMF may be derived from any known source, although there are of course sustainability advantages to using HMF derived from natural or biological sources. The HMF may be provided as part of a reactant mixture, that is, a mixture containing HMF and other components; generally this is a liquid. Such a mixture may include HMF dissolved in a solvent. The present invention allows use of less harsh solvent conditions than some prior art methods.

For example, the HMF maybe be provided in a solvent, preferably an organic solvent, for example a solvent comprising or being one or more of tetrahydrofuran (THF), water, ethanol, methanol, propanol, 1-butanol, ethyl acetate, methyltetrahydrofuran, acetonitrile and DMSO, suitably comprising one or more of tetrahydrofuran (THF), water and 1-butanol.

The solvent may in some embodiments be a single type of solvent. It may alternatively be a mixture of two different solvents, or of three different solvents, or four or more different solvents. Where it is a mixture of two or more solvents, one of those two may preferably be THF; alternatively or additionally one may preferably be water.

The solvent may in some embodiments be THF alone. In other embodiments it may be THF including some amount of water, for example up to 50% by weight of the solvent, and preferably 5-15 wt% or 5-12 wt% water in THF. A THF+water mixture, for example including 5-15 wt% water, may be preferred to enhance synthesis of BHMTHF and 1,2,6-HT.

Other components can be added to the reactant mixture, within the solvent especially. One significant possible inclusion is a metal oxide, for example CaO, which has been found to enhance or promote formation of 1,2,6-HT in the present reaction. Such an additional component may be present in an amount 0.1-5 wt%, for example 2-3 wt% in the reactant mixture.

The reactant mixture (for example HMF + solvent) may be monophasic or biphasic.

The amount of HMF included in the reactant mixture can be varied depending on the type of reactor being used and so on. Generally the reactant mixture (HMF + solvent) includes 0.01 to 99 wt%, for example 0.01 to 50 wt%, 0.1 to 10 wt% or 2 to 3 wt%, HMF. This is based on the total weight of the reactant mixture.

### Catalyst

The catalyst described herein comprises nickel (Ni) and cerium (Ce). Ideally these are supported on a third component, which may be for example a metal oxide. Such a metal oxide support may preferably be porous. With Ni and Ce loaded on the support, a porous support increases the available surface area for loading and hence reaction, increasing catalytic activity per unit volume of the catalyst material. The Ni and Ce is loaded on the surface and pore surfaces of the metal oxide.

Suitable metal oxide supports include Al₂O₃, SiO₂, CeO₂, BaO, CaO, MgO, ZrO₂, and TiO₂. Al₂O₃ and SiO₂ are preferred; of these, Al₂O₃ is most preferred and especially γ-Al₂O₃.

The Ni and Ce present in the catalyst may ideally be in the form of bimetallic NiCe. It is considered that the Ce and Ni act in concert to provide a synergistic catalysation of the present reactions. Each promotes the reaction as catalysed by the other.

The catalyst is preferably in the solid phase; this, in combination with a liquid reactant mixture as discussed above, leads to the catalyst being heterogenous. Heterogenous catalysts are easily separated from a reaction mixture. Heterogeneous catalytic systems offer substantial benefits in terms of downstream processing and costs; accordingly, the catalyst being in solid form and the reactant mixture being in liquid form is preferred.

The amount of Ni and Ce contained in the catalyst can be referred to as the loading of these components. The present catalysts do not require particularly high loadings of Ni or Ce. For example, for Ni a loading of 1-30 wt% (by weight of the catalyst as a whole, including the Ni, Ce and metal oxide if present, and any other ingredients), more preferably 2-15 wt% and most preferably 5-10 wt% is used. For Ce, a loading of 1-30 wt% (by weight of the catalyst as a whole, including the Ni, Ce and metal oxide if present, and any other ingredients), more preferably 2-20 wt% and most preferably 10-15 wt% is used.

For example the catalyst may suitably comprise 5-10 wt% Ni and 10-15 wt% Ce. Particularly suitable catalysts comprise about 10 wt% Ni and about 15 wt% Ce; or comprise about 5 wt% Ni and about 10 wt% Ce.

Aspects of the present invention are directed to catalysts as described herein. For example a catalytic material comprising a metal oxide, ideally porous, having Ni and Ce supported thereon. In some embodiments, the porous metal oxide may be selected from Al₂O₃, SiO₂, CeO₂, BaO, CaO, MgO, ZrO₂, or TiO₂, with the preferred porous metal oxide being γ-Al₂O₃. Al₂O₃ and SiO₂ may be particularly suitable in some embodiments.

For example, suitable catalysts may comprise 5-10 wt% Ni and 10-15 wt% Ce supported on either Al₂O₃ or SiO₂. Most particularly, suitable catalysts comprise about 10 wt% Ni and about 15 wt% Ce supported on Al₂O₃; or comprise about 5 wt% Ni and about 10 wt% Ce supported on either Al₂O₃ or SiO₂.

The catalyst may, as the Ni and Ce component, comprise bimetallic NiCe. This may be formed by sequential deposition of Ni and Ce (any order although preferably Ce first then Ni) onto a porous metal oxide as mentioned above.

The amount of catalyst used in the present methods is highly dependent on the reaction technique used (for example the type of reactor used). It may be from 0.1 to 10000 wt%, for example 0.1 to 400 wt% or 0.1 to 100 wt% based on the mass of HMF used in the reaction. In the examples below, which are indicative of certain preferred methods, approximately 10 wt% of the catalyst, based on the weight of HMF is used (that is, the HMF:catalyst ratio is 10:1). Accordingly, in some embodiments of the present invention (in particular those conducted in a batch reactor) the catalyst content may be 0.1 to 20 wt% compared to the weight of HMF present; that is, an HMF:catalyst ratio of from 5:1 to 1000:1.

The catalyst may be made by, for example, impregnation of a metal oxide first with a Ni containing compound (for example nickel nitrate) or a Ce containing compound (for example cerium nitrate); then calcining the resultant support; then impregnating with the other of the Ni containing compound (for example nickel nitrate) or Ce containing compound (for example cerium nitrate); then calcining the resultant support.

The calcining steps lead to the metallic deposits of Ni and Ce, and hence a bimetallic NiCe catalytic product. Those steps may be conducted by heating in air at, for example, 200 to 800°C for 1-6 hours. Preferred methods involve calcining in air each time at about 400°C for about 4 hours.

Preferably the cerium compound is added first.

Once both calcination steps have been carried out, there may be further drying (for example at 80-150°C for 1-12 hours) and calcination (for example in air at 200 to 800°C for 1-6 hours) steps.

The cerium and nickel containing compounds may be impregnated into the metal oxide as aqueous solutions containing a desired amount of nickel and cerium. The metal oxide may be immersed in such solutions; alternatively, the pore volume of the metal oxide support (where porous) may be measured for example by the BET method, and an amount of impregnation solution equal to that pore volume may be used for each of the cerium and nickel impregnation steps.

Before being used in the reaction of HMF, the catalyst may be treated by reduction in a furnace at a temperature of 200 to 800 °C, preferably between 400 - 500 °C, in a reductive atmosphere containing hydrogen (for example an atmosphere of pure hydrogen), for a time in the range 1-6 hours.

In some embodiments, where the cerium is impregnated into the metal oxide first, it may be preferable to calcine the cerium in a nitrogen atmosphere, rather than in air. Such a calcination may be at a higher temperature, for example at 600-1000°C, preferably about 800°C, for 1-6 hours. This comes before the nickel is impregnated into the metal oxide.

The inventors have found that this calcination of cerium in a nitrogen atmosphere can increase selectivity for production of BHMTHF.

### Process

The present reaction can be carried out in any type of suitable reactor: options will be apparent to those skilled in this technical field. For example, a batch reactor (e.g. a stainless steel one) or a continuous fixed bed reactor can be used.

Where a batch reactor is used, stirring may optionally be conducted during step (a). If it is, the stirring may preferably be at a speed/rate of 100-5000 rpm, preferably 400-1200 rpm. The reactor may have a "reactant" feed and a "product" feed.

Where a continuous fixed bed reactor is used, there is of course a continuous flow of reactant over the catalyst. In such a reactor, a weight hourly space velocity (= weight of reactant mixture/feed flowing through the reactor per hour, per unit weight of catalyst in the reactor) may be in the range of 0.1-10 h⁻¹.

Step (a) of the present method involves the reaction of HMF. However, after that reaction there may be further steps to extract and isolated the desired products, in particular BHMTHF, BHMF and 1,2,6-HT. Clearly, only those compounds which are actually made can be extracted.

Accordingly the present methods may include, after step (a), a step (b) of separating the catalyst from the liquid phase. This separation may be completed by, for example, one or more of filtration, sedimentation, or fixation of the catalyst. Where a continuous fixed bed reactor is used no such separation is generally necessary.

Once the liquid phase product has been obtained (either directly from step (a) or after step (b)), that liquid phase is still a mixture of reaction products. The present methods may therefore optionally include a further step (c) of isolating whichever of BHMF, BHMTHF and 1 ,2,6-HT that has/have been made from the liquid phase product, for example by evaporation of solvent and water. Of course, other methods of isolation are known to those skilled in this technical field.

### Reaction Conditions

The conditions used for step (a), the reaction of HMF, can be varied to control the final product mix obtained. There are some general preferences, though.

The atmosphere under which step (a) is carried out is a hydrogenating or reducing one. Generally it includes H₂. The H₂ content of the atmosphere may preferably be at least 50 vol%, for example at least 60 vol%, at least 70 vol%, at least 80 vol% or at least 90 vol%. In preferred embodiments the reaction atmosphere is 100 vol% (that is, pure) Hz. This can permit an efficient conversion of HMF to desired products.

The temperature of reaction used is also influential on the products obtained. In general a temperature of 100-250°C is useful; not particularly high as compared to prior art methods. A more preferred temperature range may be 150-250°C or 160-200°C for the production of BHMTHF, or 120-150°C for the production of BHMF.

The pressure of the reaction is again not particularly high. In general a pressure of greater than 0 but less than or equal to 50 MPa, for example 1-20 MPa, can be used. Preferred pressures include for example 4-10 MPa or 3-7.5 MPa. A pressure of about 5 MPa may be particularly suitable. In particular these pressures are appropriate where a pure H₂ reaction atmosphere is used.

The time for which step (a) is conducted is not particularly limited; of course, the longer it is carried out the further reactions may progress. The reaction need only be carried out until the conversion of reactant and intermediate(s) are completed, or the maximum or desired yield of desired products is reached. Suitable times for this step are 0.1 to 48 hours, more suitable 1-24 hours, for example 1-8 hours and preferably 1-6 hours, most preferably 3-6 hours.

### Selectivity

As is clear from the above, the present general reaction can form one or more of BHMTHF, BHMF and 1,2,6-HT from HMF. Which of those is formed, and in what amount, can be affected by control of reaction ingredients and certain components as explained above.

For example, to help increase the yield of 1,2,6-HT, conducting step (a) in the presence of CaO may be effective. Along with that, a temperature of 170-200°C, a shorter reaction time (for example 0.5-3 hours) and the use of a solvent which is THF + 10-15 wt% water may also be useful. General preferences set out above for other factors still apply of course.

To help increase the yield of BHMTHF, a higher reaction temperature (for example 170-220°C) and/or longer reaction time (for example 5-8 hours) may be effective.

To help increase the yield of BHMF, a lower reaction temperature (for example 120-150°C) may be effective.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the scope of the invention as defined in the appended claims.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### Examples

### Materials Used

In the Examples, the following starting materials were used:
Nitrogen and hydrogen gas (5.0, Messer, Bad Soden am Taunus, Germany), Tetrahydrofuran (>95 wt.%, EMD Millipore, USA), 1-butanol (>95 wt.%, EMD Millipore, USA), 5-hydroxymethylfurfural (>97 wt.%, Carbosynth, reference FH10853), MilliQ water, nickel (II) nitrate hexahydrate (97 wt.%, Sigma-Aldrich, St. Louis, MO, USA, reference number 72253), cerium (III) nitrate hexahydrate (99 wt.%, Sigma-Aldrich, St. Louis, MO, USA), a specific γ-alumina, Spheralite 537 (Procatalyse, France), and KIT-6 silica support (ACS Material, USA).

All these materials were used as received from the suppliers, i.e. without any additional purification.

### Analytical methods used for liquid phase analysis:

Liquid samples collected during the reaction and final reaction mixture were first filtered with 0.22µm PET syringe filters, and then directly analyzed and quantified with GC-FID and identified with GCMS. GC was equipped with a nonpolar column (ZebronTM ZB-5MS, length 60 m, diameter 0.25 mm, film thickness 0.25 µm). Injection temperature was 290 °C, injection volume 1µl and a split ratio of 80. The initial column temperature was 60 °C and held for 6.5 minutes, then increased to 160 °C with a heating rate 10 K min-1, then heated further to 290 °C with a heating rate of 15 K min-1, and held for final 5 minutes. The following retention times belongs to the suitable compounds: 2,5-bis(hydroxymethyl)furan RT =16.95 min, 2,5-bis(hydroxymethyl)tetrahydrofuran RT = 16.26 min (trans isomer) and 16.43 min (cis isomer), 1,2,6-hexanetriol RT = 18.41min.

### EXAMPLE 1

### Catalyst preparation

The preparation of the NiCe catalysts involved the impregnation of γ-Al₂O₃, specifically in this case Spheralite 537, as support using (sequential) incipient wetness impregnation method with aqueous solutions of nickel nitrate and cerium nitrate equal to the total pore volume of the support. Ceria and nickel were calcined in air at 400 °C for 4 hours after each sequential impregnation. Then samples were dried overnight at 110 °C, then calcined.

Ceria nitrate was added first, dried, calcined at the desired temperature and atmosphere, then sequentially nickel nitrate was added under the same method. Ceria and nickel were both calcined in air at 400 °C for 4 hours after each sequential impregnation.

The resultant catalyst compositions comprised 10 wt.% Ni and 15 wt.% Ce. Such a content can be notated as 10Ni 15Ce; the catalyst as a whole thus being 10Ni15Ce/γ-Al₂O₃.

Prior to the reaction, the catalyst was reduced at 400 °C under a flow of pure hydrogen for 3 hours.

### Catalytic hydrotreatment of hydroxymethylfurfural over NiCe / y-Alumina in pure THF under H₂ atmosphere

Hydrogenation tests were studied in a six parallel batch high-pressure autoclave system (Parr 5000 Series). Each batch reactor consists of a 75 mL vessel mixed with a magnetically driven stir bar and a liquid phase sampling line, which enabled collecting liquid samples over the entire reaction time. The initial reaction solvent consisted of a total of 38 g consisting THF, 1 g of 5-Hydroxymethylfurfural (HMF), and 0.1 g of reduced NiCe / γ-Alumina heterogeneous catalyst.

Reactant, solvent and catalyst(s) were weighed in appropriate amounts and placed in an autoclave vessel and sealed.

Firstly, each autoclave was flushed three times with inert N₂ atmosphere to remove any air in the vessel, then pressurized with hydrogen (5 MPa). Agitation of 1000 min⁻¹ ensured sufficient mixing for complete dispersion of the catalyst particles and gaseous phase aspiration. The reaction temperature was set to 160 °C starting at room temperature, heated up to the set temperature with a heating ramp 5 K min-'. Hydrodeoxygenation reactions was performed for 6 h once the desired reaction temperature was reached. After the desired reaction time was completed, the autoclave was rapidly cooled down. When the temperature decreased below 30 °C, the pressure was released from the autoclave before opening to collected the final liquid mixture and spent catalysts. Liquid samples were obtained throughout the entire reaction time duration to quantify the yield of liquid intermediates and products by gas chromatography and mass spectrometry (GC-FID/MS 2010 Ultra, Shimadzu, Kyoto, Japan).

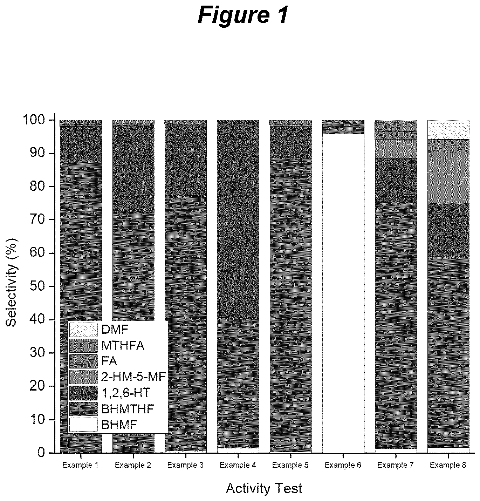

The achieved final yield of products was 88 mol.% BHMTHF and 10 mol.% of 1,2,6-hexanetriol. Figure 1 shows a summary of all products generated in the Examples described herein; Table 1 below mentions the exact amounts of the main three products of interest formed (BHMF, BHMTHF, 1 ,2,6-HT) in the Examples described herein.

### EXAMPLE 2

### Catalyst preparation

The preparation of the NiCe catalyst followed the identical process as reported in Example 1.

### Catalytic hydrotreatment of hydroxymethylfurfural over NiCe / y-Alumina in a monophasic solvent mixture of THF/water under H₂ atmosphere

The reaction proceeded as outlined above for Example 1; however, the initial reaction mixture consisted of 35 g of THF and 5 g of MilliQ water, 1 g of 5-HMF, and 0.1 g of reduced NiCe / γ-Alumina heterogeneous catalyst. The reaction operating conditions were 190 °C and 5 MPa of initial hydrogen pressure with a reaction time of 3 hours.

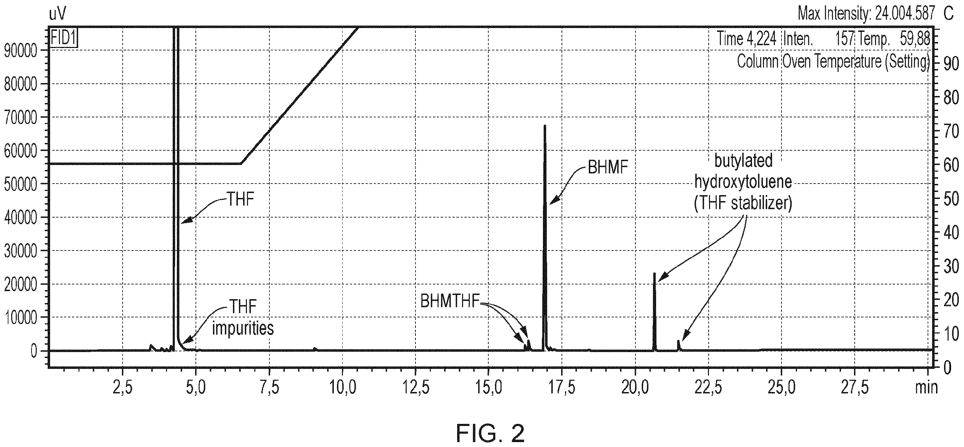

The achieved final yield of products was 27 mol.% of 1 ,2,6-hexanetriol, and 71 mol.%. Figure 2 shows the GC-FID spectrum typically obtained.

### EXAMPLE 3

### Catalyst preparation

The preparation of the NiCe catalyst followed the identical process as reported in Example 1.

### Catalytic hydrotreatment of hydroxymethylfurfural over NiCe / y-Alumina in a biphasic solvent mixture of 1-butanol/water under H₂ atmosphere

The reaction proceeded as outlined above for Example 1; however, the initial reaction solvent consisted of 35 g of 1-butanol and 5 g of MilliQ water, 1 g of 5-HMF, and 0.1 g of reduced NiCe / γ-Alumina heterogeneous catalyst. The reaction operating conditions were 190 °C, 5 MPa of initial hydrogen pressure, and a reaction time of 3 hours.

The achieved final yield of products was 22 mol.% of 1 ,2,6-hexanetriol, and 76 mol.% BHMTHF.

### EXAMPLE 4

### Catalyst preparation

The preparation of the NiCe catalyst followed the identical process as reported in Example 1.

### Catalytic hydrotreatment of hydroxymethylfurfural over NiCe / y-Alumina in monophasic solvent mixture of THF/water under H₂ atmosphere and in the presence of CaO

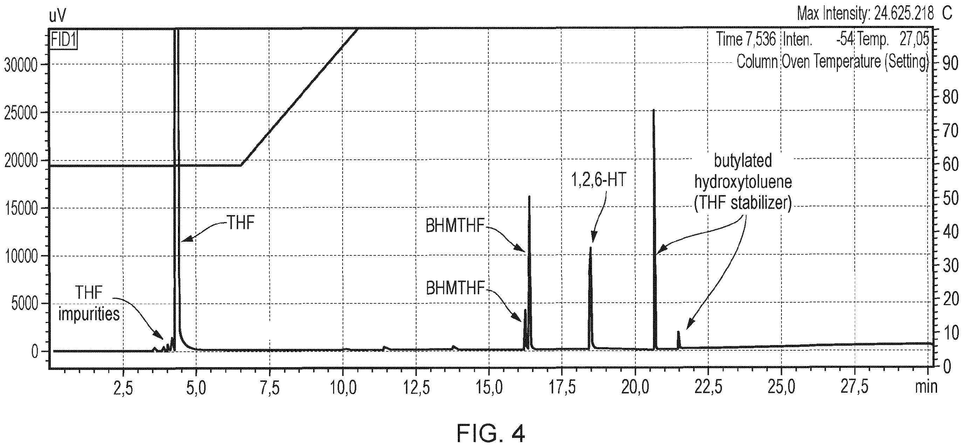

The reaction proceeded as outlined above for Example 2; however, 1 gram of CaO was added to the reaction mixture. With this, it was possible to improve the 1,2,6-hexantriol yield to 59 mol.% with a BHMTHF yield of 39 mol.% after only 1 hour at 190 °C reaction temperature. Figure 4 displays the typical GC-Fid spectrum obtained for this reaction.

### EXAMPLE 5

### Catalyst preparation

The preparation of the NiCe catalyst followed a similar process as reported in Example 1 with (sequential) incipient wetness impregnation. However, ceria was calcined in nitrogen at 800 °C for 4 hours after the initial impregnation before nickel was added. Catalyst compositions remained at 10 wt.% Ni and 15 wt.% Ce. Prior to the reaction, the catalyst was reduced at 400 °C under a flow of pure hydrogen for 3 hours.

### Catalytic hydrotreatment of hydroxymethylfurfural over NiCe/ y-Alumina in monophasic solvent mixture of THF/water under H₂ atmosphere with differing catalyst pretreatment conditions

The reaction proceeded as outlined above for Example 1; however, the initial reaction mixture consisted of 35 g of THF and 5 g of MilliQ water, 1 g of 5-HMF, and 0.1 g of reduced NiCe / γ-Alumina heterogeneous catalyst. The reaction operating conditions were 190 °C and 5 MPa of initial hydrogen pressure with a reaction time of 6 hours.

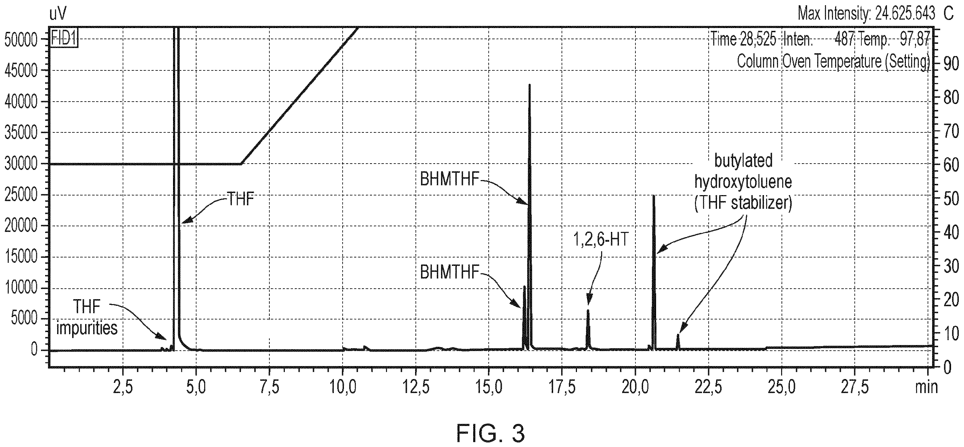

The achieved final yield of products was 10 mol.% of 1 ,2,6-hexanetriol, and 88 mol.% BHMTHF. Figure 3 displays the typical GC-Fid spectrum obtained for this reaction.

### EXAMPLE 6

### Catalyst preparation

The preparation of the NiCe catalyst followed the identical process as reported in Example 1.

### Catalytic hydrotreatment of hydroxymethylfurfural over NiCe / y-Alumina in monophasic solvent mixture of THF/water under H₂ atmospheres under lower reaction temperature

The reaction proceeded as outlined above for Example 1; however, the initial reaction mixture consisted of 35 g of THF and 5 g of MilliQ water, 1 g of 5-HMF, and 0.1 g of reduced NiCe / γ-Alumina heterogeneous catalyst. The reaction operating conditions were 140 °C and 5 MPa of initial hydrogen pressure with a reaction time of 6 hours.

The achieved final yield of products was 96 mol.% of BHMF, and 4 mol.% BHMTHF.

### EXAMPLE 7

### Catalyst preparation

The preparation of the NiCe catalyst followed the identical process as reported in Example 1, with the exception that the Ni and Ce loadings were instead 5 wt.% and 10 wt.% respectively.

### Catalytic hydrotreatment of hydroxymethylfurfural over NiCe / y-Alumina with lower loadings in pure THF under H₂ atmospheres

The reaction proceeded as outlined above for Example 1; however, the initial reaction mixture consisted of 40 g of THF, 1 g of 5-HMF, and reduced 0.1 g of NiCe / γ-Alumina heterogeneous catalyst. The reaction operating conditions were 200 °C and 5 MPa of initial hydrogen pressure with a reaction time of 6 hours.

The achieved final yield of products was 1 mol.% of BHMF, and 74 mol.% BHMTHF, and 13 mol.% 1,2,6-hexanetriol.

### EXAMPLE 8

### Catalyst preparation

The preparation of the NiCe catalyst followed the identical process as reported in Example 7, with the exception that an SiOz support was used; specifically KIT-6.

### Catalytic hydrotreatment of hydroxymethylfurfural over NiCe / SiO₂ in monophasic solvent mixture of THF/water under H₂ atmosphere

Identical reaction conditions to those reported in Example 7 were used. The achieved final yield of products was 2 mol.% BHMF, 57 mol.% BHMTHF, and 16 mol.% 1,2,6-hexanetriol.

**Table 1. Reaction conditions along with product selectivity for all Examples.**

| Example | Catalyst | Solvent | Temp. (°C) | Pressure (MPa) | Time (h) | Conv. (%) | Selectivity (%) (BHMF, BHMTHF, 1,2,6-HT) | | |
|---|---|---|---|---|---|---|---|---|---|
| 1 | 10%Ni+15%Ce on Al₂O₃ | THF | 160 | 5 | 6 | 100 | 0 | 88 | 10 |
| 2 | 10%Ni+15%Ce on Al₂O₃ | THF/water | 190 | 5 | 3 | 100 | 0 | 71 | 27 |
| 3 | 10%Ni+15%Ce on Al₂O₃ | 1-BuOH/water | 190 | 5 | 3 | 100 | 1 | 76 | 22 |
| 4 | 10%Ni+15%Ce on Al₂O₃ | THF/water + CaO | 190 | 5 | 1 | 100 | 2 | 39 | 59 |
| 5 | 10%Ni+15%Ce on Al₂O₃ (calcined at 800 °C in N₂) | THF/water | 190 | 5 | 6 | 100 | 0 | 88 | 10 |
| 6 | 10%Ni+15%Ce on Al₂O₃ | THF/water | 140 | 5 | 6 | 100 | 96 | 4 | 0 |
| 7 | 5%Ni+10%Ce on Al₂O₃ | THF | 200 | 5 | 6 | 100 | 1 | 74 | 13 |
| 8 | 5%Ni+10%Ce on SiO₂ | THF | 200 | 5 | 6 | 100 | 2 | 57 | 16 |

## Claims

1. A method of producing 2,5-bis(hydroxymethyl)furan and/or 2,5-bis(hydroxymethyl)tetrahydrofuran and/or 1,2,6-hexanetriol;
the method comprising the step of (a) contacting hydroxymethylfurfural with a catalyst comprising Ni and Ce in an atmosphere comprising Hz at a temperature in the range 100 to 250°C;
the content of Ni in the catalyst being 1-30 wt%; the content of Ce in the catalyst being 1-30 wt%.

2. A method according to claim 1, wherein the content of Ni in the catalyst is 5-10 wt% and the content of Ce in the catalyst is 10-15 wt%.

3. A method according to claim 1 or claim 2, wherein the catalyst comprises a porous metal oxide having Ni and Ce supported thereon.

4. A method according to any preceding claim, wherein the porous metal oxide is selected from Al₂O₃, SiO₂, CeO₂, BaO, CaO, MgO, ZrO₂, or TiO₂.

5. A method according to claim 4, wherein the porous metal oxide is Al₂O₃ or SiOz.

6. A method according to claim 5, wherein the porous metal oxide is γ-Al₂O₃.

7. A method according to any preceding claim, wherein the catalyst comprises bimetallic NiCe.

8. A method according to any preceding claim, wherein step (a) is conducted at a temperature in the range 120 to 200°C.

9. A method according to any preceding claim, wherein step (a) is conduced in an H₂ atmosphere.

10. A method according to any preceding claim, wherein step (a) is conducted at a pressure in the range 1-20 MPa, preferably 4-10 MPa.

11. A method according to any preceding claim, wherein step (a) is conducted for a time in the range 1-24 hours.

12. A method according to any preceding claim, wherein in step (a) hydroxymethylfurfural is present in a solvent, the solvent being one of, or a mixture of two or more of, tetrahydrofuran, 1-butanol and water.

13. A method according to claim 12, wherein the solvent comprises 0-50 wt% of water, and preferably is tetrahydrofuran mixed with 5-15 wt% water.

14. A method according to claim 12 or claim 13, wherein the solvent and hydroxymethylfurfural mixture further comprises CaO.

15. A method according to any preceding claim, further comprising a step (b) of separating the catalyst from the liquid phase.

## Patentansprüche

1. Verfahren zur Herstellung von 2,5-Bis(hydroxymethyl)furan und/oder 2,5-Bis(hydroxymethyl)tetrahydrofuran und/oder 1,2,6-Hexantriol;
wobei das Verfahren den Schritt des (a) Kontaktierens von Hydroxymethylfurfural mit einem Katalysator, der Ni und Ce umfasst, unter einer Atmosphäre, die H₂ umfasst, bei einer Temperatur im Bereich von 100 °C bis 250 °C umfasst;
wobei der Gehalt von Ni in dem Katalysator 1 bis 30 Gew.-% beträgt und der Gehalt von Ce in dem Katalysator 1 bis 30 Gew.-% beträgt.

2. Verfahren nach Anspruch 1, wobei der Gehalt von Ni in dem Katalysator 5 bis 10 Gew.-% beträgt und der Gehalt von Ce in dem Katalysator 10 bis 15 Gew.-% beträgt.

3. Verfahren nach Anspruch 1 oder Anspruch 2, wobei der Katalysator ein poröses Metalloxid mit darauf geträgertem Ni und Ce umfasst.

4. Verfahren nach einem der vorangegangenen Ansprüche, wobei das poröse Metalloxid aus Al₂O₃, SiO₂, CeO₂, BaO, CaO, MgO, ZrO₂ und TiO₂ ausgewählt ist.

5. Verfahren nach Anspruch 4, wobei das poröse Metalloxid Al₂O₃ oder SiO₂ ist.

6. Verfahren nach Anspruch 5, wobei das poröse Metalloxid γ-Al₂O₃ ist.

7. Verfahren nach einem der vorangegangenen Ansprüche, wobei der Katalysator bimetallisches NiCe umfasst.

8. Verfahren nach einem der vorangegangenen Ansprüche, wobei Schritt (a) bei einer Temperatur im Bereich von 120 °C bis 200 °C durchgeführt wird.

9. Verfahren nach einem der vorangegangenen Ansprüche, wobei Schritt (a) unter einer H₂-Atmosphäre durchgeführt wird.

10. Verfahren nach einem der vorangegangenen Ansprüche, wobei Schritt (a) bei einem Druck im Bereich von 1 bis 20 MPa, vorzugsweise 4 bis 10 MPa durchgeführt wird.

11. Verfahren nach einem der vorangegangenen Ansprüche, wobei Schritt (a) über einen Zeitraum im Bereich von 1 bis 24 h durchgeführt wird.

12. Verfahren nach einem der vorangegangenen Ansprüche, wobei in Schritt (a) Hydroxymethylfurfural in einem Lösungsmittel vorliegt, wobei das Lösungsmittel eines aus oder ein Gemisch aus zwei oder mehreren aus Tetrahydrofuran, 1-Butanol und Wasser ist.

13. Verfahren nach Anspruch 12, wobei das Lösungsmittel 0 bis 50 Gew.-% Wasser umfasst und vorzugsweise Tetrahydrofuran, gemischt mit 5 bis 15 Gew.-% Wasser ist.

14. Verfahren nach Anspruch 12 oder Anspruch 13, wobei das Gemisch aus Lösungsmittel und Hydroxymethylfurfural weiters CaO umfasst.

15. Verfahren nach einem der vorangegangenen Ansprüche, das weiters einen Schritt (b) des Abtrennens des Katalysators von der flüssigen Phase umfasst.

## Revendications

1. Procédé de production de 2,5-bis(hydroxyméthyl)furane et/ou de 2,5-bis(hydroxyméthyl)tétrahydrofurane et/ou de 1,2,6-hexanetriol ;
le procédé comprenant l'étape de (a) mise en contact d'hydroxyméthylfurfural avec un catalyseur comprenant Ni et Ce dans une atmosphère comprenant H₂ à une température dans la plage de 100 à 250°C ;
la teneur en Ni dans le catalyseur étant de 1 à 30 % en poids ; la teneur en Ce dans le catalyseur étant de 1 à 30 % en poids.

2. Procédé selon la revendication 1, dans lequel la teneur en Ni dans le catalyseur est de 5 à 10 % en poids et la teneur en Ce dans le catalyseur est de 10 à 15 % en poids.

3. Procédé selon la revendication 1 ou la revendication 2, dans lequel le catalyseur comprend un oxyde métallique poreux sur lequel sont supportés Ni et Ce.

4. Procédé selon l'une quelconque des revendications précédentes, dans lequel l'oxyde métallique poreux est choisi parmi Al₂O₃, SiO₂, CeO₂, BaO, CaO, MgO, ZrO₂ ou TiO₂.

5. Procédé selon la revendication 4, dans lequel l'oxyde métallique poreux est Al₂O₃ ou SiO₂.

6. Procédé selon la revendication 5, dans lequel l'oxyde métallique poreux est γ-Al₂O₃.

7. Procédé selon l'une quelconque des revendications précédentes, dans lequel le catalyseur comprend NiCe bimétallique.

8. Procédé selon l'une quelconque des revendications précédentes, dans lequel l'étape (a) est effectuée à une température dans la plage de 120°C à 200°C.

9. Procédé selon l'une quelconque des revendications précédentes, dans lequel l'étape (a) est effectuée dans une atmosphère de H₂.

10. Procédé selon l'une quelconque des revendications précédentes, dans lequel l'étape (a) est effectuée à une pression dans la plage de 1 à 20 MPa, de préférence de 4 à 10 MPa.

11. Procédé selon l'une quelconque des revendications précédentes, dans lequel l'étape (a) est effectuée pendant une durée dans la plage de 1 à 24 heures.

12. Procédé selon l'une quelconque des revendications précédentes, dans lequel, à l'étape (a), l'hydroxyméthylfurfural est présent dans un solvant, le solvant étant l'un parmi du tétrahydrofurane, du 1-butanol et de l'eau, ou un mélange de deux ou plus de ceux-ci.

13. Procédé selon la revendication 12, dans lequel le solvant comprend 0 à 50 % en poids d'eau, et est de préférence du tétrahydrofurane mélangé avec 5 à 15 % en poids d'eau.

14. Procédé selon la revendication 12 ou la revendication 13, dans lequel le mélange de solvant et d'hydroxyméthylfurfural comprend en outre CaO.

15. Procédé selon l'une quelconque des revendications précédentes, comprenant en outre une étape (b) de séparation du catalyseur à partir de la phase liquide.
